# EUROPEAN PATENT APPLICATION

(11) **EP 0 801 306 A1**
(43) Date of publication of application: **15.10.1997**
(21) Application number: 97105521.5
(22) Date of filing: 03.04.1997
(51) Int. Cl.: G01N 33/48, G01N 33/483, G01N 33/52, G01N 33/53, G01N 15/14, G01N 1/30, C12Q 1/68

(54) **Method for immunohistochemically quantitating co-localized molecules**

(30) Priority: 10.04.1996 US 630240
(71) Applicant: Becton Dickinson and Company, Franklin Lakes, New Jersey 07417-1880 (US)
(72) Inventor: Nauwelaers, Frans, 3191 Hever (BE); Oud, Peter, 2121 CH Bennenbrock (NL)
(74) Representative: Rambelli, Paolo

(57) **Abstract**

The present invention relates to a method for quantitating molecules co-localized in an area of a cell or a tissue. The method involves the use of combinations of staining agents which when contacted with the molecules of interest have minimal chemical and light absorbance interference of one another.

## Description

### BACKGROUND OF THE INVENTION

The present invention relates broadly to immunohistochemical analysis, and more specifically to a method for simultaneously quantitating amounts of types of molecules co-localized within a cell or tissue. A particular utilization of the invention is for the simultaneous quantitation of nuclear antigens and DNA to allow for simultaneous quantitative immunoploidy and quantitative nuclear antigen analysis.

Double staining techniques have been developed in order to identify two or more different components simultaneously and are widely used for example in the field of biology. A limited number of dual staining methods are known which allow for the application of simultaneous two-color analytical techniques.

EP-A2-0 317 139 [17.11.1987] discloses a method of marking selected cells in a population and for measuring the DNA masses of preselected classes of cells (an immunoploidy analysis) employing a dual-staining method consisting of staining of the cell population with an alkaline phosphatase technique applying a monoclonal antibody against a specific cytoplasmic antigen with fast red dye as the chromogen, and then staining the DNA via a Feulgen staining procedure using Thionin. The related U.S. Pat. No. 5,134,662 also details the method of performing a two-color analysis whereby a Feulgen staining technique for quantitative immunoploidy (QIP) analysis which classifies cells based on a blue color in the nucleus for DNA and a red chromogen in the cytoplasm for monoclonal antibodies specific against cytoplasmic antigens.

U.S. Patent No. 5,134,662 discloses two other dual-staining techniques for performing two-color analysis: an ionic staining technique with methyl or ethyl green is used to mark the nucleus and a diaminobenzidene (using a peroxidase-monoclonal antibody) or red chromogen (using an alkaline phosphatase-monoclonal antibody) is used to combine with a specific component in the nucleus. The latter method is employed for quantitative proliferation indexing, i.e. the measure of proliferation in cells or tissues by using immunoperoxidase staining techniques with an antibody to proliferating cells.

The above-cited staining techniques provide optical enhancement of molecules in either two distinct cellular components, specifically the nucleus and the cytoplasm and allows quantification, or permits localization of one cellular compartment and quantification of a macromolecule located within that cellular compartment. Thus, these known methods do not permit the simultaneous quantitation of co-localized molecules, that is molecules in the same area of a tissue or cell whether that be the cytoplasm, nucleus, mitochondria, Golgi bodies, lysosomes or other organelles.

### SUMMARY OF THE INVENTION

To address this inability in the art and other problems of staining co-localized molecules such as chemical interference of the staining agents with one another, the present invention relates to a method for simultaneously quantitating multiple co-localized molecules in a cell or tissue by: fixing the cell or tissue, staining a first type of molecule in the cell or tissue, staining other types of molecules co-localized with the first type of molecule with a stain which has minimal absorption at the wavelength at which the stained first type of molecule absorbs, and measuring the amount of light absorbance of stained co-localized molecules.

### DETAILED DESCRIPTION OF THE INVENTION

As stated above, the present invention relates to a method for quantitating types of molecules which are co-localized in a cell or tissue. Co-localization of molecules herein refers to the location of types of molecules within the same organelle or other area of a tissue or a cell, for example the cell nucleus, mitochondria, Golgi bodies, cytoplasm, lysosomes or other organelles.

The utilization of staining agents which when contacted with the co-localized molecules of interest have minimal absorption at the absorbance wavelength of the other stained co-localized molecules permits the quantitation of multiple co-localized molecules from a single fixed cell preparation. Minimal absorption as used herein refers, in a functional manner, to a level of absorption which does not interfere with the measurement of light absorbance of the other stained co-localized molecules. Such minimal absorbance may be accomplished by the selection of particular staining agents and types of co-localized molecules to be contacted by the staining agents. It is generally preferred that stained co-localized molecules do not exhibit greater than about 30% absorbance of any of the other stained co-localized molecules. When using computerized image analysis systems to perform the method of the present invention software adjustments may be made to enhance the images to aid in quantitating co-localized molecules.

The staining agents which are effective for the method of the present invention include CAS Red (available from Becton Dickinson Cellular Imaging Systems) in combination with various alkaline phosphatase based detection systems such as the Pathway AP kit also available from Becton Dickinson Cellular Imaging Systems, and various thionin based stains applied after Feulgen type reactions which are particularly useful for DNA staining. Such DNA staining techniques are taught in EP-A2-O 317 139 and U.S. Patent No. 5,134,662, and are effectively employed with the CAS DNA staining kit available from Becton Dickinson Cellular Imaging Systems.

The staining agents selected for use in the method of the present invention should also have minimal chemical interference with one another as they will stain co-localized molecules. The types of co-localized molecules which would be stained include any co-localized cellular markers of interest such as DNA, estrogen receptor (ER), progesterone receptor (PR), MIB-1, p53, retinoblastoma (Rb) antigen, PCNA, cyclins and KI67, etc. Although many combinations of staining agents and co-localized molecules may be utilized in the method of the present invention, the method has been found to be particularly effective when nuclear markers such as MIB-1, ER and/or PR are immunologically stained with a fast red staining agent such as CAS Red, and DNA is stained in a conventional manner such as with a Feulgen type reaction with thionin staining. When these types of molecules were stained in this manner, the wavelengths selected for absorbance are 500 nm and between 580 nm and 620 nm with 620 nm providing optimal results, that is optimal visualization with minimal interference or absorption by the other stained co-localized molecules. The CAS Red stained MIB-1, ER and/or PR nuclear antigens are quantitated with usage of the 500 nm filter with no measurable interference from the blue thionin stained DNA. Conversely, the blue thionin stained DNA is quantitated with use of the 580 nm or 620 nm filter with no measurable interference from the CAS Red stained other co-localized nuclear antigens (MIB-1, ER and/or PR).

Before staining the co-localized molecules, the cells or tissue containing the molecules are fixed using conventional cell or tissue fixative techniques. Such conventional cell and tissue fixative techniques include acetone fixation (5 minutes at - 20°C followed by 5 minutes at room temperature) and 2% paraformaldehyde in phosphate buffered saline (PBS) (5 minutes at 4°C) followed by methanol (10 minutes at -20°C).

The determination of light absorbance of the stained co-localized molecules may be accomplished by any known means. However, an automated cellular imaging system such as the Cell Analysis Systems 200 (CAS 200) instrument or the Discovery instrument, both of which are available from Becton Dickinson Cellular Imaging Systems is preferred.

The following examples illustrate specific embodiments of the invention described in this document. As would be apparent to skilled artisans, various changes and modifications are possible and are contemplated within the scope of the invention described.

### EXAMPLE 1

### Quantitation of DNA and MIB-1

### Cells and Antibody

The test cells used for this Example were: MCF 7 cells grown on glass slides until about 50% confluency, and the primary antibody used was MIB-1 (Immunotech) at dilutions of 1:50, 1:100 and 1:200.

### Cell Fixation and Staining

The slides were immediately fixed according to 3 different fixation procedures (see below) and immunostained according to the insert of the Pathway AP kit (Becton Dickinson Cellular Imaging Systems). CAS Red was made according to the insert of the CAS Red kit (Becton Dickinson Cellular Imaging Systems) and developed for 10 minutes.

The slides were additionally fixed overnight in neutral buffered formalin. DNA staining was done using CAS DNA staining kit (Becton Dickinson Cellular Imaging Systems). Although the solvability of the CAS Red chromogen is poor in alcohol, a too long (more than 6 minutes) dehydration can remove some of the chromogen.

The three fixation techniques used were:
- acetone (5 minutes at -20° C, 5 minutes at room temperature);
- 2% paraformaldehyde in PBS (5 minutes at 4°C) followed by methanol (10 minutes at -20°C); and
- Zamboni (10 minutes at room temperature).

The Zamboni cell fixation technique proved to be inadequate, probably because the membranes of the (intact) cells were not made permeable. The acetone and paraformaldehyde/methanol fixations gave identical acceptable results.

The optimal dilution for MIB-1 antibody (visually determined) was 1:200.

### Discovery Instrument Measurements

1000 cells per slide were measured with the 25x objective in the high magnification mode (total magnification 50x), using the black and white camera. DNA was measured with the 620 nm filter and the CAS Red stained antigen with the 500 nm filter. During measurement images of the measured nuclei were recorded with the color camera.

A scatterplot was created in which the IOD of the DNA stain was plotted on the X-axis and the IOD of the MIB-1 stain on the Y-axis. Regions were set around the MIB-1 positive and negative cells. This latter population could be discriminated on the basis of the negative control (no MIB-1), that was measured beforehand. The distribution of the positive cells is very similar to that found with flow cytometry measurements: an increasing MIB-1 content is seen as the cells travel from G1 through the S-phase to G2M. 95.7% of the cells are MIB-1 positive.

More specific data of the DNA and MIB-1 histograms of the different fractions are shown in the Tables following the other Examples.

### CAS 200 Measurements

300 cells were measured with the "Quantitative DNA Analysis" program version 3.0. Positive and negative cells were visually selected and put in different classes. The individual histograms were analyzed and the G1 and G2M phases determined using the "area ABCD option". The most significant data of all histograms are presented in the Tables following the other Examples. Again, as with the DISCOVERY measurements, CAS 200 measurements showed a slightly higher DNA value for the G1 and G2M peaks of the MIB-1 positive cells and virtually the same for the negative ones, as compared to the control.

### EXAMPLE 2

### Quantitation of DNA and ER

### Cells and Antibody

As in Example 1, MCF 7 cells grown on glass slides to about 50% confluency were used. The primary antibody was Estrogen Receptor antibody 1D5 (Dako) at dilutions of 1:100, 1:200 and 1:400.

### Cell Fixation and Staining

The same staining and cell fixation techniques used in Example 1 were used in this Example.

As in Example 1, the Zamboni cell fixation technique was not adequate. For ER, the paraformaldehyde/methanol technique was optimal.

Also, the optimal dilution for ER antibody was 1:100.

### Discovery Instrument Measurements

The same procedures followed in Example 1 were also used in this Example. Based on the negative control, an ER negative and ER positive fraction were discriminated. The distribution of the positive cells was different from that found for DNA-MIB-1. About two thirds (65.9%) of the cells were ER positive. More specific data of the DNA and ER histograms from the Discovery instrument are shown in the Tables following the next Example.

### CAS 200 Measurements

The same procedures followed in Example 1 were also used in this Example. Virtually the same results as in Example 1 were found in this Example as compared to ER control. More specific data of the DNA and ER histograms from the CAS 200 instrument are shown in the Tables following the next Example.

### EXAMPLE 3

### Quantitation of DNA and PR

### Cells and Antibody

As in Example 1, MCF 7 cells grown on glass slides to about 50% confluency were used. The primary antibody was PR 33 (Becton Dickinson Cellular Imaging Systems) at dilutions of 1:100, 1:200 and 1:400.

### Cell Fixation and Staining

The same staining and cell fixation techniques used in Example 1 were used in this Example.

As in Example 1, the Zamboni cell fixation technique was not adequate. For PR, the paraformaldehyde/methanol technique was optimal.

Also, the optimal dilution for PR antibody was 1:200.

### Discovery Instrument Measurements

The same procedures followed in Example 1 were also used in this Example. The scatterplot of DNA and PR was similar to that for DNA and ER in Example 2, however in this case about one quarter (22.4%) of the cells were PR positive. More specific data of the DNA and PR histograms from the Discovery instrument are shown in the Tables below.

### CAS 200 Measurements

The same procedures followed in Example 1 were also used in this Example. Virtually the same results as in Example 1 were found in this Example as compared to PR control. More specific data of the DNA and PR histograms from the CAS 200 instrument are shown in the Tables below.

**TABLE 1**

| **DISCOVERY DNA** | | | | | | | |
|---|---|---|---|---|---|---|---|
| **FIXATION** | **IMMUNO** | | **G1 PEAK** | | **G2M PEAK** | | **IOD G2M/G1** |
| | | | **IOD** | **CV** | **IOD** | **CV** | |
| Acetone | control | all cells | 475 | 2.88 | 958 | 2.72 | 2.02 |
| | MIB-1 | pos. cells | 523 | 5.78 | 1071 | 3.38 | 2.05 |
| | | neg. cells | 487 | 3.84 | - | - | - |
| Paraform/Me thanol | control | all cells | 474 | 4.26 | 948 | 3.55 | 2.00 |
| | ER | pos. cells | 486 | 3.34 | 979 | 2.66 | 2.01 |
| | | neg. cells | 482 | 3.84 | 990 | 1.37 | 2.05 |
| | PR | pos. cells | 488 | 3.23 | 963 | 2.93 | 1.97 |
| | | neg. cells | 474 | 3.31 | 943 | 3.43 | 1.99 |

**TABLE 2**

| **DISCOVERY Immunostain** | | | |
|---|---|---|---|
| FIXATION | IMMUNO | | IOD immunostain ±sd |
| Acetone | control | all cells | 25±43 |
| | MIB-1 | pos. cells | 1436±651 |
| Paraform/Methanol | control | all cells | 51±55 |
| | ER | pos. cells | 439±190 |
| | PR | pos. cells | 640±340 |

For the DNA-MIB-1 stained cells the IOD values of the G1 and G2M peaks of the positive cells increased slightly, as compared to the control. The value of both peaks increase in a similar fashion, as can be concluded from the G2M/G1 ratio, that stayed constant. Of the negative cells no major increase was seen in the G1 peak position (no G2M cells, as expected, could be found here) Furthermore, the cv of the G1 peak of the positive cells doubled as compared to the control.

DNA combined with ER or PR hardly showed significant differences in the different features of the DNA-histogram of the different cell populations, as compared with the control.

The slight increase in the IOD and cv of the G1 of the MIB-1 positive cells, as compared to ER and PR, can be explained by the more intense immunostaining as can be seen in the Immunostain Table (Table 2). The average IOD of 1436 is much higher than that of ER (439) and PR (640). Thus, although visually no interference of the immunostaining is seen at the wavelength of the DNA measurement a software correction for this small interference should be made. This will also be beneficial for future other nuclear immunostainings of which the intensities are still unknown.

**TABLE 3**

| **CAS 200 DNA** | | | | | | | |
|---|---|---|---|---|---|---|---|
| **FIXATION** | **IMMUNO** | | **G1 PEAK** | | **G2M PEAK** | | **IOD G2M/G1** |
| | | | | **IOD** | **CV** | **IOD** | **CV** |
| Acetone | Control | all cells | 1.75 | 2.38 | 3.61 | 2.08 | 2.06 |
| | MIB-1 | pos. cells | 1.91 | 4.07 | 3.99 | 2.77 | 2.09 |
| | | neg. cells | 1.78 | 4.13 | - | - | |
| Paraform/Me thanol | control | all cells | 1.78 | 2.11 | 3.61 | 1.50 | 2.03 |
| | ER | pos. cells | 1.80 | 3.35 | 3.58 | 1.85 | 1.99 |
| | | neg. cells | 1.76 | 3.27 | 3.56 | 2.46 | 2.02 |
| | PR | pos. cells | 1.86 | 4.25 | 3.72 | 4.19 | 2.00 |
| | | neg. cells | 1.77 | 2.84 | 3.66 | 1.48 | 2.07 |

While the invention has been described with some specificity modifications apparent to those of ordinary skill in the art may be made without departing from the scope of the invention. Various features of the invention are set forth in the following claims.

## Claims

1. A method for quantitating multiple co-localized molecules in a cell or a tissue comprising the steps of:
fixing the cell or tissue;
staining a first type of molecule in the cell or tissue;
staining other types of molecules co-localized with the first type of molecule with a stain which has minimal absorption at the wavelength at which the stained first type of molecule absorbs; and
measuring the amount of light absorbance of stained co-localized molecules.

2. The method of claim 1 wherein the molecules are co-localized in the nucleus of the cell.

3. The method of claim 1 wherein the first type of molecule is selected from the group consisting of ER, PR, MIB-1, p53, Rb, PCNA, cyclins and KI67.

4. The method of claim 1 wherein the first type of molecule is immunocytochemically stained.

5. The method of claim 4 wherein the first type of molecule is immunocytochemically stained with a fast red dye.

6. The method of claim 5 wherein the fast red dye is CAS Red.

7. The method of claim 1 wherein the staining of other types of molecules is accomplished with a Feulgen type reaction with Thionin staining.
